## Europäisches Patentamt
## European Patent Office
## Office européen des brevets

(11) Publication number: **0 254 486 B1**

(12) **EUROPEAN PATENT SPECIFICATION**

(45) Date of publication of the patent specification: **10.10.90**

(51) Int. Cl.⁵: **C07C 59/105, C07C 51/43**

(21) Application number: **87306312.7**

(22) Date of filing: **16.07.87**

(54) Preparation of metal gluconates.

(30) Priority: **18.07.86 FI 862988**

(43) Date of publication of application: **27.01.88 Bulletin 88/4**

(45) Publication of the grant of the patent: **10.10.90 Bulletin 90/41**

(84) Designated Contracting States: **AT BE CH DE ES FR GB GR IT LI LU NL SE**

(56) References cited:
**US-A- 1 942 660**
**US-A- 2 701 811**
**US-A- 3 907 640**

(73) Proprietor: **Stabra AG, Baarerstrasse 2, CH-6301 Zug(CH)**

(72) Inventor: **Heikkila, Heikki O., Lansituulentie 7, Espoo(FI)**
Inventor: **Nurmi, Juha V., Lansituulentie 7, Espoo(FI)**

(74) Representative: **Jones, Ian et al, POLLAK MERCER & TENCH High Holborn House 52-54 High Holborn, London WC1V 6RY(GB)**

## Description

The invention relates to the preparation of a metal gluconate from an aqueous solution.

Metal gluconates, and in particular, calcium gluconate and zinc gluconate, are used as additives in the food and animal feed industries. Although the water solubility of calcium gluconate is relatively low, it has not been feasible, to date, to prepare a crystalline calcium gluconate product on an industrial scale, nor has it been feasible to prepare a crystalline zinc gluconate product on an industrial scale.

Currently available calcium gluconate is either an agglomerated product or a dried and milled product prepared by precipitating the salt and drying the crystal mass which is formed. For example, in one prior art method, a calcium gluconate solution is evaporated to 15-20% by weight by precipitating the salt at temperatures ranging from 0°C-25°C. The resulting mass is separated by centrifugation, washed with water and dried at 60°C-80°C in a fluidized bed drier. However, centrifugation under these circumstances does not effectively remove the mother liquor from the crystal mass. In addition, the drying process is complicated due to formation of dust and lumps. Drying produces hydrated calcium gluconate crystals which can be milled and dried to form an anhydrous powder which is, however, not as desirable as anhydrous crystals.

The prior art also discloses methods for precipitation of metal gluconates from organic solvents. For example, Hungarian Patent No. 29928 described a method for the preparation of tetragonic anhydrous crystals by precipitation from an organic solvent. When water is evaporated at 80°C - 110°C from a solution of calcium gluconate in the organic solvent the salt forms a precipitate which can be separated and dried. This method is not, however, suitable for industrial scale production.

Similarly, Melson and Pickering (Aust J. Chem (1968) PD 2889-3) describe a method for the production of zinc gluconate trihydrate by precipitation from organic solvents. The method is not suitable for industrial scale due to problems in separating the small crystals from the solution and also because handling of organic solvents in large scale is always problematic.

Prior art methods for the production of dry zinc gluconates utilize spray drying from water solutions. The spray dried zinc gluconate product is, of course, impure, because the drying process does not provide any purification of the product.

It has now been discovered, however, that crystalline metal gluconates, including calcium gluconate and zinc gluconate, can be produced on an industrial scale by precipitation from a seeded, supersaturated aqueous solution, the crystallization being carried out in two steps.

The present invention accordingly provides a method for the efficient crystallization of metal gluconates including calcium gluconate and zinc gluconate from an aqueous solution, which can be used on an industrial scale. Crystal yield is relatively high, and the crystals are of uniform size, and easy to separate by centrifugation and to wash and dry. Practising the present invention results in very pure, uniform crystals which are easy to handle, in contrast to prior art methods.

The crystalline calcium gluconate product is characterized by a uniform crystal size with a length of over 20 um (with an average length of about 100 to 200 um), a width of over 10 um (with an average width of about 15 to about 20 um) and a height of over 5 um (with an average height of about 10 to about 15 um). The crystalline zinc gluconate product is characterized by a uniform crystal size with an average length over 20 um (with an average length of about 50-150 um) width over 3 um (with an average width of about 5-20 um) and height over 1 um (with an average height of about 3-10um).

Suitable raw materials for the preparation of crystalline calcium gluconate include all calcium gluconate solutions, including those containing sodium gluconate and other impurities; purification of the sodium gluconate to remove impurities, however, speeds up the crystallization rate.

In practicing the present invention, crystallization is carried out using an aqueous feed solution at a temperature in excess of about 50°C, preferably about 60°C to about 95°C. The feed solution must be slightly acidic, with a pH of about 4 to about 7. The aqueous feed solution is concentrated by evaporation to a degree of supersaturation with respect to the metal gluconate present of about 1.3 to about 2.3. For the preparation of calcium gluconate, the preferable degree of supersaturation is about 1.7 to about 2.3; for the preparation of zinc gluconate, the preferable degree of supersaturation is about 1.5 to about 1.8.

A relatively large number of seed crystals of sufficient size (approximately 20 - 200 um long, 2 - 10 um wide and 2 - 5 um high) are prepared by evaporation centrifugation and are introduced into and dispersed throughout the feed solution. The seed crystals can be generated by a separate crystallization process. For example, a highly supersaturated, purified (with the zinc gluconate comprising over 99% by weight of the dry substance) solution will crystallize spontaneously, forming microcrystals which can be separated and used as seed crystals in a bench scale crystallization to obtain seed crystals for the final large scale crystallizations.

It is also possible to use crystals from a previous crystallization, a process known as "footing".

The first crystallization step comprises evaporation crystallization at high temperatures (above 50°C) and reduced pressure. A high degree of supersaturation with respect to the metal gluconate is maintained by the addition of feed solution. In the second step, the resulting crystals and solution are transferred to a suitable agitation device where the crystals continue to grow. During this step the tempera-

.ture is maintained at above 50°C, any temperature decrease is limited to about 20°C, with a more particularly preferred decrease limited to about 3°C to about 10°C.

Following crystallization, the produced crystals are recovered by centrifugation, filtration or any other convenient solids-liquid separation expedient.

Preferred embodiments of the invention are described below in detail.

The rate of crystallization usually involves two actions: (a) the rate of formulation of new crystals; a nucleation, either in a clear solution or one containing solids, and (b) the rate of precipitation of solute on crystals already present, usually called crystal growth. The present invention utilizes methods involving the latter.

The deposition of a solid from a solution onto a crystal can take place only if there is a state of imbalance with a driving force. This means that the solution must be supersaturated with respect to crystals of the size on which depositions is to occur before the crystals can grow by deposition from the solution.

The degree of supersaturation at a given temperature and composition of the solution is calculated from the following formula:

$$ S = \frac{pl}{(100 - pl)} \cdot \frac{(100 - ps)}{ps} $$

where S = degree of supersaturation
pl = the dry substance of the solution (weight %)
ps = the dry substance of a saturated solution (weight %)

The starting material for the present purposes is an aqueous solution of metal gluconate, acidified to a pH of between about 4 to about 7. The aqueous solution is concentrated by evaporation to a degree of supersaturation with respect to the metal gluconate of about 1.3 to about 2.3 to form a feed solution.

Seed crystals of sufficient size (approximately 200 - 200 um long, 2 - 10 um wide and 1 - 5 um high) are prepared by evaporation centrifugation and are introduced into and dispersed throughout the feed solution. According to the present invention, the subsequent crystallization of the metal gluconate is preferably carried out in two steps.

The first step is an evaporation crystallization at a temperature of about 70°C to about 95°C; for the preparation of calcium gluconate, the preferred temperature is about 80°C to about 90°C and for the preparation of zinc gluconate the preferred temperature is about 60°C to about 70°C. The solution has a degree of supersaturation of about 1.3 - 2.3 (for the preparation of calcium gluconate the solution has preferably about 1.7 - 2.0 and for the preparation of zinc gluconate the preparation has about 1.5 to about 1.8) which is maintained during this step by the addition of feed solution. This step generally lasts about 3 - 25 hours during which crystals have grown to 5% - 40% of the total mass.

In the second step, the resulting crystals and solution are transferred to a suitable agitation device where the crystal mass is mixed for about 5 - 70 hours (preferably about 10 - 30 hours). During this step a relatively high temperature must be maintained. Any lowering of the temperature during the second step (the agitation temperature) must not exceed a drop of 20°C and should preferably be less than about 3°C.

The resulting crystals can be separated by centrifugation or other suitable means and washed with water. The washed crystals are dried in warm air, for example, in a conventional rotating drier. During centrifugation and drying the long brittle crystals break so that the final product is made up of crystals 20 - 300 um long (with an average length of about 100 - 200 um), 10 - 50 um wide (with an average width of about 15 - 30 um) and 5 - 30 um high (with an average height of about 10 - 15 um). The crystals are chemically durable and can be dried to obtain anhydrous metal gluconates.

A part of the crystal mass can be left in the crystallizer for "footing" the next crystallization batch, i.e., the "left over" crystals can be used as seed crystals.

This invention is illustrated further by the following examples.

<u>Preparation of seed crystals: calcium gluconate</u>

1 kg of glucono-delta-lactone was dissolved in 10 liters of water at 80°C. 200 g calcium hydroxide was added suspended in water (30% by weight) until the pH was 5.1. The solution was filtrated and evaporated at 70°C and 700 millibar for 32 hours. The crystals were separated by centrifugation and dried at 80°C - 90°C for approximately 12 hours. These crystals were used as seed crystals in the method described in Example 1.

<u>Example 1: Crystallization of Calcium Gluconate</u>

66 kg of glucono-delta-lactone ("GDL") and 66 kg of sodium gluconate were dissolved in 800 liters of water at 80°C. Approximately 11 kg of calcium hydroxide (technical grade, provided by Lohja Co) was added as a 30% water suspension until the pH of the solution was about 4.7. The solution was filtrated and diluted to about 14.4% by weight of dry substance.

3

The feed solution was concentrated by evaporation at about 70°C and 710 millibar to 400 liters in an agitated evaporating crystallizer. The resulting supersaturated solution containing 15.8 % by weight of dry substance was then seeded with 30 grams of anhydrous calcium gluconate crystals as prepared above, which were suspended in 100 ml isopropanol. The evaporation continued for 28 hours at 70°C while maintaining the degree of supersaturation at 1.3 by the addition of feed solution. The mass was then transferred to a 400 liter crystallizer where it was kept agitated for 16 hours at a temperature greater than 55°C.

The resulting crystals were separated by centrifugation and washed with water (ratio of water to crystal mass approximately 1:1). The washed crystals were then dried with warm air (at about 90°C) which totally removed the water. The resulting crystals were generally about 20 - 250 um long (principally between about 80 and 200 um long) 10 - 40 um wide (principally between about 15 and 25 wide) and 3 - 30 um high (principally between about 5 and 10 um high). The crystal yield was about 60% of the calcium gluconate added.

Example 2: Crystallization of Calcium Gluconate

7.85 tons of glucono-delta-lactone was dissolved in 70 cubic meters of water at 80°C. Approximately 1.7 tons of calcium hydroxide (technical grade) was added as a 30% water suspension until the pH was 5.5 and the solution was filtrated. The dry substance of the solution was 11.5% by weight.

The solution was evaporated at 90°C and 760 millibar until the supersaturation was 1.7 (dry substance 23% by weight). The solution was then seeded with 3.0 kg calcium gluconate crystals suspended in 10 liters of isopropanol.

The evaporation was continued at 90°C for 20 hours. The resulting crystals were transferred to a crystallizer for continued crystallization for approximately 20 hours. The total dry substance of the mass was 35% of added calcium gluconate at the end of this step and the temperature was 87°C. The degree of supersaturation was about 1.0.

The resulting crystals were then separated by centrifugation in a conventional basket centrifugal machine equipped with a net and a cloth (between the net and the basket) to recover small crystals. The crystals were washed in water (water to mass ratio of approximately 1:3) and dried in a conventional rotating drier (from A. Ahlström Co) with warm air (at about 90°C). During the centrifugation and drying the long brittle crystals broke to form a product where the crystals were about 20 - 200 um long (principally about 100 to about 200 um long), about 10 - 50 um wide (principally about 15 to about 30 um wide) and about 5 - 30 um high (principally about 10 to about 15 um).

The crystal yield was about 55% of the calcium gluconate.

Composition of the Product

|  | Calcium Gluconate Product from Example 2 | Calcium Gluconate Prepared from Prior Arth Method |
|---|---|---|
| Assay (EDTA) | 99.7% | 97.0% |
| Water | 0.0 – 0.2% | over 1% |
| IR absorbance (cm – 1) | 1620(s) 1420(s) 1370(m)1330(m) 1270(s)1200(m) 1080(s) 1010(s) 930(m) 900(m) 860(m) | 1600(s) 1370 (s) 1300(m) 1230(m) 1200 (m) 1100(s) 1040(s) 970(m) 880(m) 820(m, sh) |
| Form | Crystals | Powder |

Example 3: Crystallization of Calcium Gluconate

7.85 tons of glucono-delta-lactone was dissolved at 80°C in 70 cubic meters of water. 1.7 tons of calcium hydroxide was added as a 30% water suspension until the pH was 5.5 and the solution was filtrated. The dry substance content of the solution was 11.5%. The solution was transferred to an evaporation crystallizer which contained a 2500 kg crystal mass from a previous crystallization as a "foot".

The solution was evaporated at 90°C for approximately 18 hours and then the main part of the mass (approximately 95%) was transferred to a crystallizer where the mass was agitated for another 13 hours. At the end of the process the mass contained 35% dry substance and the temperature was 86°C. The degree of supersaturation was about 1.0.

The crystals were separated by centrifugation (Landwerk Buckau R Wolf) equipped with a net and cloth as in Example 2. The crystals were subsequently dried in a rotating drier. The crystal yield was about 50% of the calcium gluconate added.

### Preparation of seed crystals: zinc gluconate

1 kg of glucono-delta-lactone was dissolved in 10 liters of water at 50°C. 230 g of zinc oxide was added as a 30% water suspension until the pH of the solution was about 4.2. The solution was filtrated and then evaporated at reduced pressure at a temperature of 50°C for 11 hours. The formed crystals were separated from the solution by centrifugation and dried for about 12 hours at a temperature of about 80°C - 90°C. These crystals were used as seed crystals.

### Example 4: Crystallization of zinc gluconate

150 kg glucono-delta-lactone was dissolved in 450 kg water at 60°C. 34 kg of zonc gluconate (from Kuusankoski Oy) were added as a 30% water suspension until the pH of the solution was 5.4 and the solution was filtered. The dry substance content of the filtered solution was 24.1% by weight.

The solution was evaporated at a pressure of 200 millilbar until the degree of supersaturation with respect to zinc gluconate was 1.9 (dry substance 47.6 % by weight). 40 g of seed crystals (as prepared above) were added suspended in 150 ml of saturated zinc gluconate water solution.

The evaporation was continued at 60°C for 3 hours allowing the crystals to grow to long needle-like crystals. Thereafter, the main part of the mass was transferred to a crystallizer, where the solution was agitated for another 30 hours. At the end of the crystallization, the temperature was about 55 C and the degree of supersaturation with respect to zinc gluconate was close to 1. The total dry substance content of the mass was 48.3 % by weight.

The crystals were separated in a conventional basket centrifugal machine where a cloth was applied on the basket to catch small crystals. The crystals were washed with water (approximately 30% by weight of the mass) and dried with warm air at a temperature of about 90°C in a rotating drier. During centrifugation and drying, the long brittle crystals broke to a final length of about 20 - 200mm (with an average length of about 50 - 150mm), width of 3 - 30 mm (with an average width of about 5 - 20 mm) and height of 1 - 20 mm (with an average height of about 3 - 10 mm).

The yield was 50% of the zinc gluconate added.

An analysis of the zinc gluconate trihydrate crystals compared to granule zinc gluconate indicates the following:

| | Zinc gluconate prepared by the present invention | Prior Art Zinc Gluconate |
|---|---|---|
| Appearance | Crystalline | Powder |
| Infra-red absorbance | 1590(s), 1410(s, sh) | |
| 1590(m), 1410(b), (cm − 1) | 1310(s), 1240(s) | |
| 1170(s), 1060(m, sh) | 1090(s), 1040(s) 980(m), 870(s) | 870(m) |

### Example 5: Crystallization of Zinc Gluconate

150 kg of glucono-delta-lactone and 34.5 kg of zinc oxide were dissolved in water as in Example 4. The pH was buffered to about 6.5 and the solution filtrated. The dry substance content of the solution was 34.8% by weight.

The solution was evaporated at reduced pressure at 300 millibar and 70°C until the degree of supersaturation with respect to zinc gluconate was 1.8 (dry substance 54.4 % by weight). 180 g zinc gluconate crystals in 680 ml saturated water solution were added as seed crystals and the evaporation continued at 70°C for 3 hours. Thereafter the main part of the mass was transferred to a crystallizer where the crystals were allowed to grow for another 60 hours. At the end of the crystallization, the dry substance of the mass was 52.8% by weight and the temperature was 60°C. The supersaturation was close to 1.

The crystals were separated from the solution by centrifugation and washed with water as in Example 4, with the yield being 45% of the zinc gluconate.

It is also possible to use the methods described herein for the production of other crystalline metal gluconates.

The foregoing description is to be understood as illustrative of the invention and not as limiting its scope as defined by the appended claims.

## Claims

1. A method of recovering a metal gluconate from an aqueous metal gluconate solution, the method comprising the steps of:

providing a supersaturated solution of the metal gluconate in an aqueous solution having a pH between about 4 and about 7, the solution having a degree of supersaturation with respect to the metal gluconate of at least about 1.3;

introducing seed crystals into the supersaturated solution;

removing water from the solution at a temperature in excess of 50°C to crystallize the dissolved metal gluconate while maintaining a degree of supersaturation of at least about 1.3;

agitating the resulting crystals to further crystallize dissolved metal gluconate; and

recovering the crystallized metal gluconate.

2. A method as claimed in claim 1 wherein the step of providing the supersaturated solution of the metal gluconate comprises the steps of preparing an aqueous metal gluconate solution with the pH of about 4 to about 7 and supersaturating the feed solution by evaporation to the degree of supersaturation with respect to the metal gluconate of at least 1.3.

3. A method as claimed in claim 1 or 2 wherein the metal gluconate is calcium gluconate.

4. A method as claimed in claim 1, 2 or 3 wherein the degree of supersaturation is in the range of about 1.3 to about 2.3.

5. A method as claimed in claim 4 wherein the degree of supersaturation is in the range of about 1.7 to about 2.0.

6. A method as claimed in any preceding claim wherein the crystallization temperature is between about 70°C and about 95°C.

7. A method as claimed in claim 6 wherein the crystallization temperature is between about 80°C to about 90°C.

8. A method as claimed in claim 1 or 2 wherein the metal gluconate is zinc gluconate.

9. A method as claimed in claim 1, 2 or 8 wherein the degree of supersaturation is in the range of about 1.3 to about 2.0.

10. A method as claimed in claim 9 wherein the degree of supersaturation is in the range of about 1.5 to about 1.8.

11. A method as claimed in claim 1, 2, 8, 9 or 10 wherein the crystallization temperature is between about 50°C and about 80°C.

12. A method as claimed in claim 11 wherein the crystallization temperature is between about 60°C and about 70°C.

13. A method as claimed in claim 1, 2, 8, 9 or 10 wherein the crystallization temperature is between about 70°C to about 95°C.

14. A method as claimed in any preceding claim wherein the resulting crystals are dried to produce anhydrous metal gluconate.

15. A method as claimed in any preceding claim wherein the crystallized metal gluconate is recovered by centrifugation.

**Patentansprüche**

1. Verfahren zum Rückgewinnen eines Metallgluconats aus einer wässrigen Metallgluconatlösung, welches Verfahren die Schritte umfaßt:

Bilden einer übersättigten Lösung des Metallgluconats in einer wässrigen Lösung mit einem pH-Wert zwischen etwa 4 und etwa 7, wobei die Lösung ein Maß an Übersättigung bezüglich des Metallgluconats von wenigstens etwa 1,3 hat,

Einführen von Impfkristallen in die übersättigte Lösung,

Entfernen des Wassers von der Lösung bei einer Temperatur über 50°C, um das gelöste Metallgluconat auszukristallisieren, während ein Maß an Übersättigung von wenigstens etwa 1,3 beibehalten wird,

Rühren der sich ergebenden Kristalle, um das gelöste Metallgluconat weiter auszukristallisieren und Rückgewinnen des auskristallisierten Metallgluconats.

2. Verfahren nach Anspruch 1, bei dem der Verfahrensschritt der Bildung der übersättigten Lösung des Metallgluconats die Schritte der Bildung einer wässrigen Metallgluconatlösung mit dem pH-Wert von etwa 4 bis etwa 7 und der Übersättigung der gelieferten Lösung durch Verdampfung auf ein Maß an Übersättigung bezüglich des Metallgluconats von wenigstens 1,3 umfaßt.

3. Verfahren nach Anspruch 1 oder 2, bei dem das Metallgluconat Kalziumgluconat ist.

4. Verfahren nach Anspruch 1, 2 oder 3, bei dem das Maß an Übersättigung im Bereich von etwa 1,3 bis etwa 2,3 liegt.

5. Verfahren nach Anspruch 4, bei dem das Maß an Übersättigung im Bereich von etwa 1,7 bis etwa 2,0 liegt.

6. Verfahren nach einem der vorhergehenden Ansprüche, bei dem die Kristallisationstemperatur zwischen etwa 70°C und etwa 95°C liegt.

7. Verfahren nach Anspruch 6, bei dem die Kristallisationstemperatur zwischen etwa 80°C und etwa 90°C liegt.

8. Verfahren nach Anspruch 1 oder 2, bei dem das Metallgluconat Zinkgluconat ist.

9. Verfahren nach Anspruch 1, 2 oder 8, bei dem das Maß an Übersättigung im Bereich von etwa 1,3 bis etwa 2,0 liegt.

10. Verfahren nach Anspruch 9, bei dem das Maß an Übersättigung im Bereich von etwa 1,5 bis etwa 1,8 liegt.

11. Verfahren nach Anspruch 1, 2, 8, 9 oder 10, bei dem die Kristallisationstemperatur zwischen etwa 50°C und etwa 80°C liegt.

12. Verfahren nach Anspruch 11, bei dem die Kristallisationstemperatur zwischen etwa 60°C und etwa 70°C liegt.

13. Verfahren nach Anspruch 1, 2, 8, 9 oder 10, bei dem die Kristallisationstemperatur zwischen etwa 70°C und etwa 95°C liegt.

14. Verfahren nach einem der vorherigen Ansprüche, bei dem die sich ergebenden Kristalle getrocknet werden, um ein wasserfreies Metallgluconat zu erzeugen.

15. Verfahren nach einem der vorhergehenden Ansprüchen, bei dem das kristallisierte Metallgluconat durch Zentrifugieren rückgewonnen wird.

**Revendications**

1. Un procédé pour récupérer un gluconate métallique à partir d'une solution aqueuse de gluconate métallique, le procédé comprenant les étapes suivantes:
on fournit une solution sursaturée du gluconate métallique dans une solution aqueuse ayant un pH compris entre environ 4 et environ 7, la solution ayant un degré de sursaturation par rapport au gluconate métallique d'au moins environ 1,3;
on introduit des germes cristallins dans la solution sursaturée;
on élimine l'eau de la solution à une température de plus de 50°C pour cristalliser le gluconate métallique dissous, tout en maintenant un degré de sursaturation d'au moins environ 1,3;
on agite les cristaux résultants pour cristalliser encore du gluconate métallique dissous; et
on récupère le gluconate métallique cristallisé.

2. Un procédé selon la revendication 1, dans lequel l'étape de fourniture de la solution sursaturée du gluconate métallique comprend les étapes de préparation d'une solution aqueuse de gluconate métallique d'un pH d'environ 4 à environ 7 et sursaturation de la solution d'alimentation par évaporation à un degré de sursaturation d'au moins 1,3 par rapport au gluconate métallique.

3. Un procédé selon la revendication 1 ou 2, dans lequel le gluconate métallique est le gluconate de calcium.

4. Un procédé selon la revendication 1, 2 ou 3, dans lequel le degré sursaturation est dans la gamme d'environ 1,3 à environ 2,3.

5. Un procédé selon la revendication 4, dans lequel le degré de sursaturation est dans la gamme d'environ 1,7 à environ 2,0.

6. Un procédé selon l'une quelconque des revendications précédentes, dans lequel la température de cristallisation est comprise entre environ 70 et environ 95°C.

7. Un procédé selon la revendication 6, dans lequel la température de cristallisation est comprise entre environ 80 et environ 90°C.

8. Un procédé selon la revendication 1 ou 2, dans lequel le gluconate métallique est le gluconate de zinc.

9. Un procédé selon la revendication 1, 2 ou 8, dans lequel le degré de sursaturation est dans la gamme d'environ 1,3 à environ 2,0.

10. Un procédé selon la revendication 9, dans lequel le degré de sursaturation est dans la gamme d'environ 1,5 à environ 1,8.

11. Un procédé selon la revendication 1, 2, 8, 9 ou 10, dans lequel la température de cristallisation est comprise entre environ 50 et environ 80°C.

12. Un procédé selon la revendication 11, dans lequel la température de cristallisation est comprise entre environ 60 et environ 70°C.

13. Un procédé selon la revendication 1, 2, 8, 9 ou 10, dans lequel la température de cristallisation est comprise entre environ 70 et environ 95°C.

14. Un procédé selon l'une quelconque des revendications précédentes, dans lequel les cristaux résultants sont séchés pour produire le gluconate métallique anhydre.

15. Un procédé selon l'une quelconque des revendications précédentes, dans lequel le gluconate métallique cristallisé est récupéré par centrifugation.